# FASCICULE DE BREVET EUROPEEN

(11) **EP 1 411 865 B1**
(45) Date de publication et mention de la délivrance du brevet: **26.08.2009**
(21) Numéro de dépôt: 02791513.1
(22) Date de dépôt: 30.07.2002
(51) Int. Cl.: A61F 2/24

(54) **ENSEMBLE PERMETTANT LA MISE EN PLACE D'UNE VALVE PROTHETIQUE DANS UN CONDUIT CORPOREL**
ANORDNUNG ZUM EINSETZEN EINER VENTILPROTHESE IN EIN KÖRPERGEFÄSS
ASSEMBLY FOR SETTING A VALVE PROSTHESIS IN A CORPOREAL DUCT

(30) Priorité: 31.07.2001 FR 0110281
(43) Date de publication de la demande: 28.04.2004
(73) Titulaire: CoreValve, Inc., Irvine, CA 92618 (US)
(72) Inventeur: CoreValve, Inc., Irvine, CA 92618 (US)
(74) Mandataire: Jeannet, Olivier
(86) Numéro de dépôt international: PCT/FR2002/002745
(87) Numéro de publication internationale: WO 2003/011195

(56) Documents cités:
- EP-A- 1 057 460
- WO-A-00/47139
- WO-A-99/30637
- FR-A- 2 815 844
- US-A- 5 851 232
- US-A- 5 855 601
- US-A- 6 042 607

## Description

La présente invention concerne un ensemble permettant la mise en place d'une valve prothétique dans un conduit corporel, notamment une valve cardiaque et en particulier une valve aortique.

Les documents WO 91/17720, WO 98/29057 et EP 1 057 460 décrivent chacun un ensemble de ce type, comprenant :
- la valve prothétique à implanter ;
- une armature radialement expansible, dite "stent", propre, à l'état expansé, à prendre appui contre la paroi du conduit corporel à équiper, cette prise d'appui permettant d'immobiliser ce stent par rapport à cette paroi ; et
- des moyens de fixation de la valve au stent.

La mise en place du stent permet ainsi le montage de la valve dans le conduit corporel, éliminant la nécessité d'un abord par l'extérieur et donc d'une intervention chirurgicale directe.

Cette technique toutefois a pour inconvénients essentiels d'induire un risque d'endommagement de la valve par le ballonnet utilisé pour réaliser l'expansion du stent, et de limiter la force d'expansion qu'il est possible d'imprimer au stent. Cette limitation a une répercussion sur l'ancrage du stent, rendant possible un déplacement dudit ensemble. Cette limitation a également une répercussion sur l'étanchéité du stent au niveau de l'anneau valvulaire, qui est particulièrement affectée lorsque des zones calcifiées confèrent à l'anneau valvulaire une forme irrégulière et/ou une certaine rigidité.

L'expansion d'un ballonnet peut également entraîner un endommagement du conduit corporel, particulièrement lorsque celui-ci est un vaisseau sanguin.

L'utilisation d'un stent auto expansible n'est guère possible, un tel stent n'ayant pas une force radiale d'appui à même de permettre un ancrage suffisant de l'ensemble.

De plus, le conduit corporel à équiper peut, au niveau du site d'implantation, ne pas présenter une section transversale parfaitement circulaire, notamment lorsque la valve native est conservée et que cette valve, ou l'anneau valvulaire, comprend des zones calcifiées. Quel que soit le degré d'expansion du stent, la forme circulaire de ce stent peut alors ne pas être adaptée à l'anatomie spécifique du site d'implantation. Il peut en résulter un défaut d'étanchéité de la valve implantée.

En outre, le stent présente une certaine rigidité, qui induit une rigidité du cathéter d'implantation. Cette rigidité peut rendre difficile la progression de ce cathéter jusqu'au site d'implantation.

Un autre inconvénient de la technique antérieure est de lier directement les commissures des valvules au stent. Il en résulte qu'une expansion du stent, et donc de la valve, différente de celle prévue peut entraîner une mauvaise coaptation des valvules et donc un fonctionnement défectueux de la valve. Le stent doit par conséquent faire l'objet d'une expansion prédéterminée qui empêche, ou rend difficile, l'adaptation de ce stent à la variabilité anatomique.

La technique antérieure a également pour inconvénients, en cas d'implantation d'une valve aortique, d'induire un risque de bouchage des ostia coronaires.

La présente invention vise à remédier à ces différents inconvénients.

L'ensemble qu'elle concerne comprend, de manière connue en soi, la valve prothétique à implanter et un support recevant cette valve, la valve et le support étant conformés de manière à pouvoir adopter une position de contraction radiale, qui permet leur insertion dans le conduit corporel à équiper à l'aide de moyens d'insertion / déploiement, et une position de déploiement radial, dans laquelle le support est immobilisé par rapport à la paroi du conduit corporel à équiper. Le document US-A-6 042 607 décrit un ensemble correspondant au préambule de la revendication 1.

Selon l'invention, le support comprend :
- au moins une portion tubulaire en matériau souple faiblement étirable dans le sens circonférentiel, qui présente, dans sa position de déploiement, une dimension dans le sens circonférentiel correspondant sensiblement à la dimension dans le sens circonférentiel de l'emplacement du conduit corporel en regard duquel cette portion tubulaire doit être implantée ;
- des moyens de fixation de cette portion tubulaire à la paroi du conduit corporel, et
- une pluralité d'éléments allongés d'armature, disposés sur la circonférence de ladite portion tubulaire et reliés à cette portion tubulaire de manière indépendante les uns des autres ; la valve est reliée au moins partiellement à ces éléments allongés d'armature, notamment au niveau des commissures de ses valvules, et ces éléments allongés d'armature forment conjointement, en position déployée, une structure ayant un diamètre prédéterminé qui assure le déploiement adéquat de cette valve.

Le support selon l'invention n'est ainsi pas formé par un stent expansible prenant appui par frottements contre la paroi du conduit corporel à équiper, mais est formé (i) par au moins une portion tubulaire souple faiblement étirable circonférenciellement, pourvue de moyens de fixation à la paroi du conduit corporel, et (ii) par une structure de montage de la valve, déployée par cette ou ces portions tubulaires.

Ces dernières font seules l'objet d'un déploiement actif, réalisé notamment au moyen d'un ou plusieurs ballonnets gonflables, au niveau de leurs zones pourvues desdits moyens de fixation ; les valvules de la valve sont reliées auxdits éléments allongés d'armature et sont donc placées sur une portion du support n'ayant pas à être sollicitée par les moyens de déploiement, de sorte que le risque de détérioration de ces valvules par ces moyens de déploiement est éliminé.

Le déploiement de la ou des portions tubulaires ne requiert pas une force radiale aussi importante que celle nécessaire au déploiement d'un stent, et la faible étirabilité, de cette ou ces portions tubulaires au-delà de leur position de déploiement permet de supprimer les risques de détérioration du conduit corporel résultant de l'utilisation de ballonnets gonflables.

La structure souple de la ou des portions tubulaires leur permet en outre de parfaitement s'adapter à l'anatomie spécifique du site d'implantation, particulièrement lorsque celui-ci n'est pas circulaire en section transversale du fait de la présence de zones calcifiées.

Le support a globalement une rigidité longitudinale nettement plus faible que celle d'un stent, ce qui facilite notablement la progression du cathéter d'implantation jusqu'au site d'implantation.

Les commissures des valvules ne sont pas reliées à une zone expansible, et la structure formée par lesdits éléments allongés d'armature assure le déploiement adéquat de ces valvules.

La valve peut être réalisée en tissu biologique ou en matériau synthétique comme par exemple un polymère. Elle peut présenter un anneau de base permettant sa liaison étanche, notamment par suture, à ladite portion tubulaire ou à l'une desdites portions tubulaires.

Ladite portion tubulaire, ou une paroi périphérique que comprend la valve, peut présenter des ouvertures latérales de manière à permettre un écoulement naturel du sang vers les ostia coronaires, sans risque de stagnation.

De préférence, ladite ou lesdites portions tubulaires présentent au moins une chambre périphérique gonflable, pouvant être gonflée de manière à former un joint assurant l'étanchéité entre cette portion tubulaire et la paroi du conduit corporel à équiper.

La zone proximale de la portion tubulaire, ou de la portion tubulaire proximale en cas de pluralité de portions tubulaires, peut présenter une forme tronconique adaptée à prendre appui contre la paroi du ventricule.

La ou les portions tubulaires peuvent être constituées par un matériau biologique ou synthétique, notamment un polymère ou un tissu de fibres connues sous la dénomination "dacron".

Lesdits moyens de fixation sont de préférence constitués par des agrafes d'ancrage présentant des branches d'ancrage, ces branches d'ancrage venant s'insérer dans la paroi du conduit corporel à équiper en fin de déploiement de cette ou ces portions tubulaires.

Ces agrafes peuvent être plastiquement déformables ; des moyens peuvent alors être prévus pour déployer les branches d'ancrage de ces agrafes de manière non radiale, notamment en oblique ou selon une trajectoire courbe, afin de renforcer la résistance de l'ancrage obtenu.

Au moins une de ces agrafes peut être en un matériau à mémoire de forme tel que l'alliage de nickel et de titane connu sous la dénomination "NITINOL". Une telle agrafe peut alors adopter avant implantation une configuration d'insertion, dans laquelle ses branches d'ancrage sont orientées sensiblement radialement par rapport à la portion tubulaire pour pouvoir être insérées dans la paroi du conduit, et une configuration d'ancrage, dans laquelle ces mêmes branches d'ancrage s'orientent de manière non radiale et/ou se recourbent afin d'assurer l'ancrage des agrafes dans la paroi du conduit corporel à équiper.

Les moyens de fixation peuvent également comprendre une colle biologique, pouvant être contenue dans des vésicules ruptibles placées sur la face radialement externe de la ou des portions tubulaires.

Ces vésicules se rompent lors de leur écrasement entre la ou les portions tubulaires et la paroi du conduit corporel, et permettent ainsi de libérer la colle.

Chaque élément allongé d'armature présente avantageusement une forme courbe et est disposé avec son côté convexe tourné radialement vers l'intérieur du support, de telle sorte que ce support présente, au niveau de sa zone médiane, un diamètre inférieur par rapport à celui qu'il présente au niveau de ses extrémités axiales.

Ce diamètre inférieur permet d'éliminer tout risque de recouvrement des ostia coronaires.

Selon une forme de réalisation préférée de l'invention, le support comprend :
- deux portions tubulaires telles que précitées, et
- l'une des extrémités desdits éléments allongés d'armature est reliée à l'une de ces portions tubulaires tandis que l'autre extrémité de ces éléments allongés d'armature est reliée à l'autre portion tubulaire.

Le déploiement de ces deux portions tubulaires assure un parfait déploiement des éléments allongés d'armature.

Selon une autre forme de réalisation possible de l'invention, le support comprend une portion tubulaire unique s'étendant sur l'ensemble du support, à la paroi de laquelle sont reliés lesdits éléments allongés d'armature.

Cette portion tubulaire unique peut présenter les ouvertures latérales précitées en regard des ostia coronaires.

Selon encore une autre forme de réalisation possible de l'invention, le support comprend une portion tubulaire unique, de laquelle font saillie, sur une extrémité axiale de cette portion tubulaire, lesdits éléments allongés d'armature.

Ce support peut notamment permettre l'implantation d'une valve mitrale.

Pour sa bonne compréhension, l'invention est à nouveau décrite ci-dessous en référence au dessin schématique annexé représentant, à titre d'exemple non limitatif, une forme de réalisation préférée de l'ensemble qu'elle concerne.

La figure 1 en est une vue de côté avec coupe partielle, à l'état déployé et avant implantation, dans le cas de l'implantation d'une valve aortique ;
la figure 2 en est une vue de côté en cours de positionnement dans une aorte et avant déploiement, alors qu'il est placé sur un instrument permettant son positionnement et son déploiement ;
la figure 3 en est une vue partielle, à échelle agrandie, avant ancrage à la paroi aortique ;
la figure 4 en est une vue similaire à la figure 2, en cours de déploiement ;
la figure 5 en est une vue similaire à la figure 3 après ancrage à la paroi aortique ;
la figure 6 en est une vue similaire à la figure 1 après implantation, ledit instrument de positionnement et de déploiement ayant été retiré ;
les figures 7 et 8 sont des vues dudit ensemble similaires à la figure 6 selon deux variantes de réalisation, ledit instrument de positionnement et de déploiement étant représenté schématiquement sur la figure 8 ;
les figures 9A, 10A, 11A, 12A, 13A et 14A sont des vues en coupe d'une palette que comprend ledit ensemble et d'une agrafe que comporte cette palette, avant venue de l'agrafe en position d'ancrage ;
les figures 9B, 10B, 11 B, 12B, 13B et 14B sont des vues correspondant respectivement aux figures 9A, 10A, 11 A, 12A, 13A et 14A, après venue de l'agrafe en position d'ancrage ;
la figure 13C est une vue en coupe selon la ligne XIIIC-XIIIC de la figure 13B ;
les figures 15 et 16 sont des vues en perspective d'autres variantes de réalisation d'une agrafe ;
la figure 17 est une vue de côté d'encore une autre variante de réalisation d'une agrafe ;
la figure 18 est une vue de côté d'une autre forme de réalisation dudit instrument de positionnement et de déploiement, en cours de positionnement dudit ensemble ;
la figure 19 est une vue de cet instrument similaire à la figure 18, en cours l'implantation de cet ensemble ;
la figure 20 est une vue de cet instrument en coupe selon la ligne XX-XX de la figure 19 ;
les figures 21 et 22 sont des vues en coupe longitudinale de trois autres formes de réalisation de ce même instrument, alors qu'il est placé dans une aorte, la figure 21 montrant également ledit ensemble en cours d'implantation, et
la figure 23 est une vue dudit ensemble, dans le cas de l'implantation d'une valve mitrale.

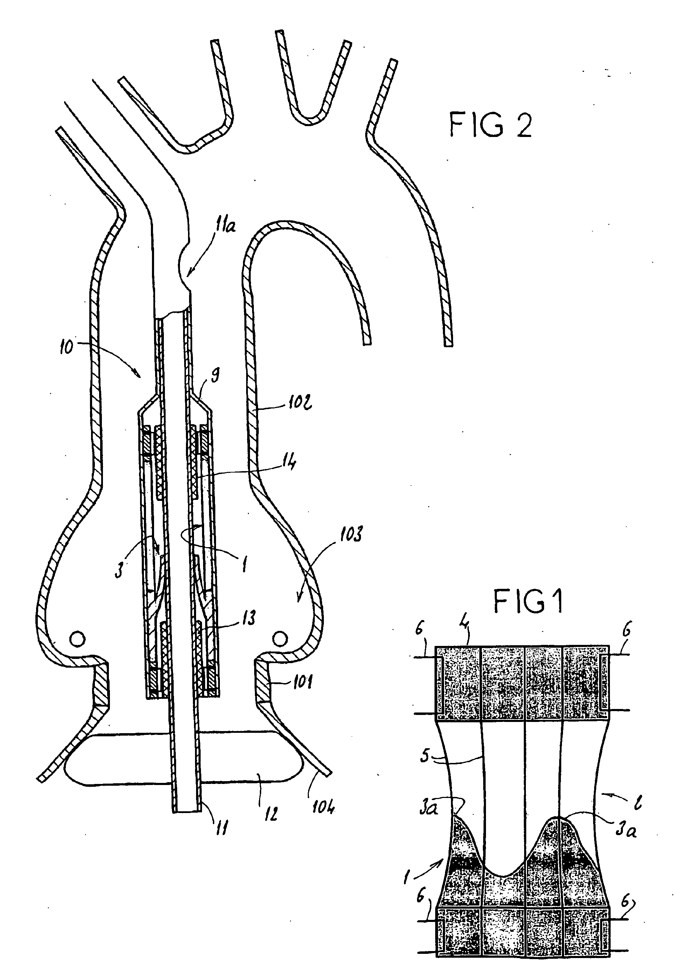

La figure 1 représente un ensemble 1 permettant la mise en place d'une valve aortique prothétique dans une aorte.

Cet ensemble 1 comprend un support 2 sur lequel est montée cette valve aortique prothétique 3.

Le support 2 comprend deux portions tubulaires d'extrémité 4 et une série d'éléments allongés d'armature 5.

Les portions tubulaires 4 sont en un matériau souple faiblement étirable dans le sens circonférenciel de ces portions tubulaires 4. Elles peuvent notamment être réalisées en polymère ou en un tissu de fibres connues sous la dénomination "dacron", ou encore en tissu biologique comme le péricarde.

Le diamètre de ces portions tubulaires 4 correspond sensiblement au diamètre des emplacements de l'aorte en regard desquels ces portions tubulaires 4 sont destinées à être implantées, c'est-à-dire, ainsi que le montrent les figures 2 à 6, respectivement l'anneau valvulaire natif 101 et la paroi 102 de l'aorte s'étendant au-delà des ostia coronaires 103.

Chaque élément allongé d'armature 5 est réalisé en un matériau relativement rigide, notamment en un matériau métallique. Il présente une portion centrale allongée courbe 5a et deux palettes d'extrémités 5b, par lesquelles il est relié aux portions tubulaires 4 respectives.

Comme le montre la figure 1, ces éléments 5 sont régulièrement répartis sur la périphérie des portions tubulaires 4 et sont disposés avec leur côté convexe tourné radialement vers l'intérieur du support 2. Ce dernier présente ainsi, au niveau de sa zone médiane, un diamètre inférieur à celui qu'il présente au niveau de ses extrémités axiales.

Chaque palette 5b est intégrée à la portion tubulaire 4 correspondante et est fixée à celle-ci notamment par des sutures. Elle comprend un ou plusieurs trous traversants disposés radialement, en particulier deux trous dans l'exemple représenté, dans lesquels sont engagées les branches latérales d'ancrage d'une agrafe 6.

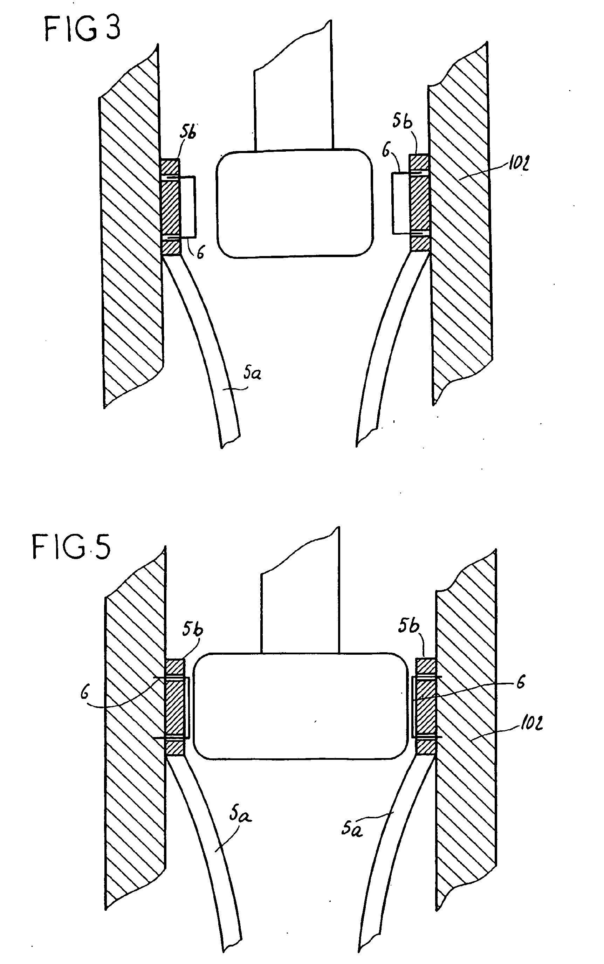

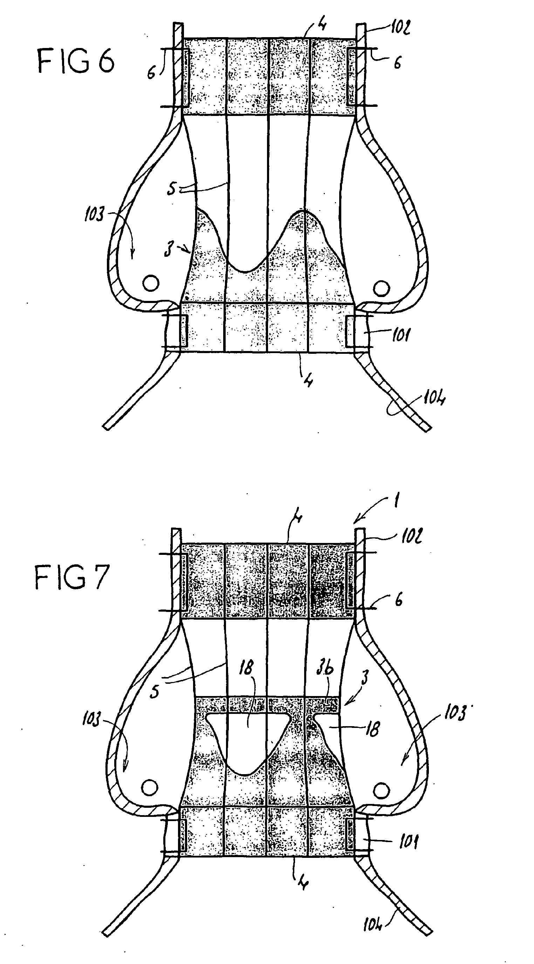

Comme le montrent les figures 1 et 3, ces branches latérales sont situées, avant implantation, en retrait des orifices radialement extérieurs de ces trous, de sorte que l'agrafe 6 ne fait pas saillie au-delà de la face radialement extérieure de la portion tubulaire 4 mais fait saillie radialement vers l'intérieur de cette portion tubulaire 4. Ces trous sont ajustés auxdites branches latérales de sorte que l'agrafe 6 est retenue dans ces trous par frottements. Ces branches peuvent toutefois coulisser dans ces trous vers une position d'ancrage représentée sur les figures 5 et 6, lorsqu'un ballon gonflable vient porter contre la branche médiane de l'agrafe 6.

La valve 3 ne fait pas partie en elle-même de l'invention et n'est donc pas particulièrement détaillée. Elle peut être réalisée en tissu biologique ou en matériau synthétique, notamment en un polymère, et présente un anneau de base relié à la portion tubulaire 4 proximale notamment par suture et des valvules dont les commissures 3a sont reliées aux portions 5a adjacentes des éléments 5.

La liaison de l'anneau de base à la portion 4 proximale assure l'étanchéité de la valve 3 entre cet anneau et cette portion tubulaire, et la liaison des commissures 3a aux éléments 5 permet le déploiement des valvules lorsque le déploiement du support 2 est réalisé.

L'instrument 10 de positionnement et de déploiement de l'ensemble 1 comprend, ainsi que cela apparaît sur la figure 2, un cathéter 11 d'écoulement du sang le temps de l'implantation de l'ensemble 1, trois ballonnets 12, 13, 14 et une gaine 9 de maintien des portions 4 et des éléments 5 dans une position de contraction.

Le cathéter 11 comprend une ouverture distale 11a d'écoulement du sang au-delà de l'ensemble 1 le temps de l'implantation. Il peut éventuellement comprendre une pompe facilitant cet écoulement.

Le ballonnet proximal 12 est tronconique et est dimensionné pour prendre appui contre la paroi évasée 104 du ventricule lorsqu'il est gonflé. Il permet ainsi, par suite de sa venue en appui contre cette paroi 104, de positionner précisément l'instrument 10 et l'ensemble 1 par rapport au site d'implantation, nonobstant les battements du coeur, et bloque le passage du sang de manière à le canaliser à l'intérieur du cathéter 11.

Le ballonnet 13 s'étend en regard de l'anneau de base de la valve 3. Il permet le déploiement de la portion 4 proximale et de cet anneau de base.

Le ballonnet distal 14 s'étend quant à lui en regard de la portion 4 distale, dont il permet le déploiement.

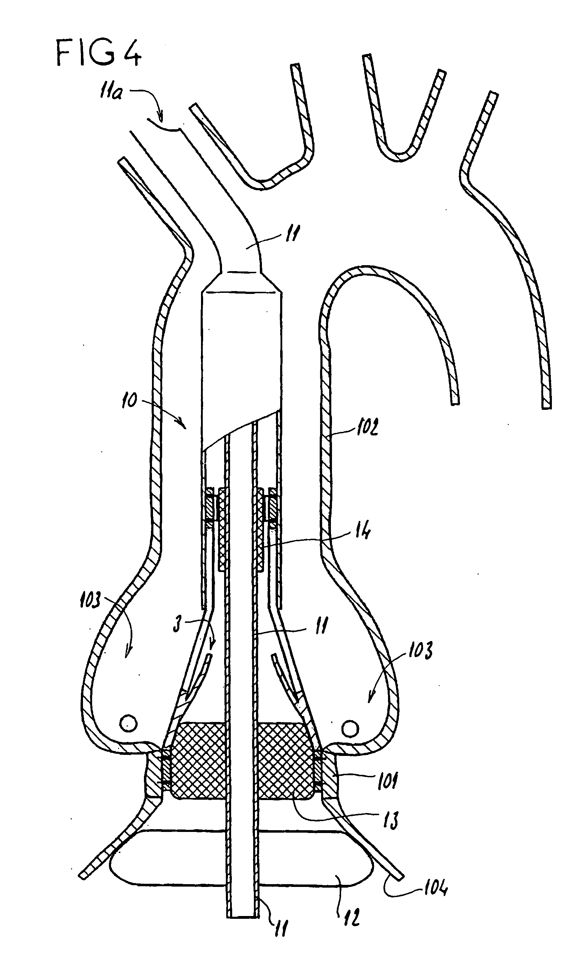

La gaine 9 est coulissante sur le cathéter 11 et permet, par son retrait, de libérer l'ensemble 1. Comme cela apparaît sur la figure 4, le ballonnet 13 est gonflé alors que la gaine 9 recouvre encore la portion 4 distale. Le gonflage du ballonnet 13 est réalisé jusqu'à plaquer la portion 4 contre l'anneau 101 puis appuyer contre les branches médianes des agrafes 6 afin de réaliser le coulissement des agrafes 6 dans les trous des palettes 5b et insérer ainsi les branches latérales de ces agrafes 6 dans cet anneau 101.

Une fois l'ancrage de la portion 4 proximale réalisé, la gaine 9 est reculée de manière à libérer la portion 4 distale, qui est déployée et ancrée au moyen du ballonnet 14, de la même manière que précitée.

Le déploiement des portions tubulaires 4 réalise le déploiement des éléments 5 et donc de la valve 3.

La structure souple des portions 4 permet à ces dernières de s'adapter à l'anatomie spécifique du site d'implantation, particulièrement lorsque la valve native est conservée et/ou que cette valve ou l'anneau 101 présente des zones calcifiées.

Les éléments 5 permettent d'assurer le déploiement adéquat des valvules quelle que soit la forme des portions tubulaires 4 après implantation, et les espaces latéraux que délimitent ces éléments 5 entre eux permettent un large écoulement du sang vers les ostia coronaires 103.

Les agrafes 6 permettent quant à elles, de par leur nombre, d'assurer une parfaite immobilisation de l'ensemble 1 dans le site d'implantation.

La figure 7 montre un ensemble 1 dans lequel la valve 3 présente une paroi périphérique 3b. Cette paroi 3b est reliée aux éléments 5 et présente des ouvertures latérales 18, aménagées entre les valvules, qui permettent l'écoulement du sang vers les ostia coronaires 103.

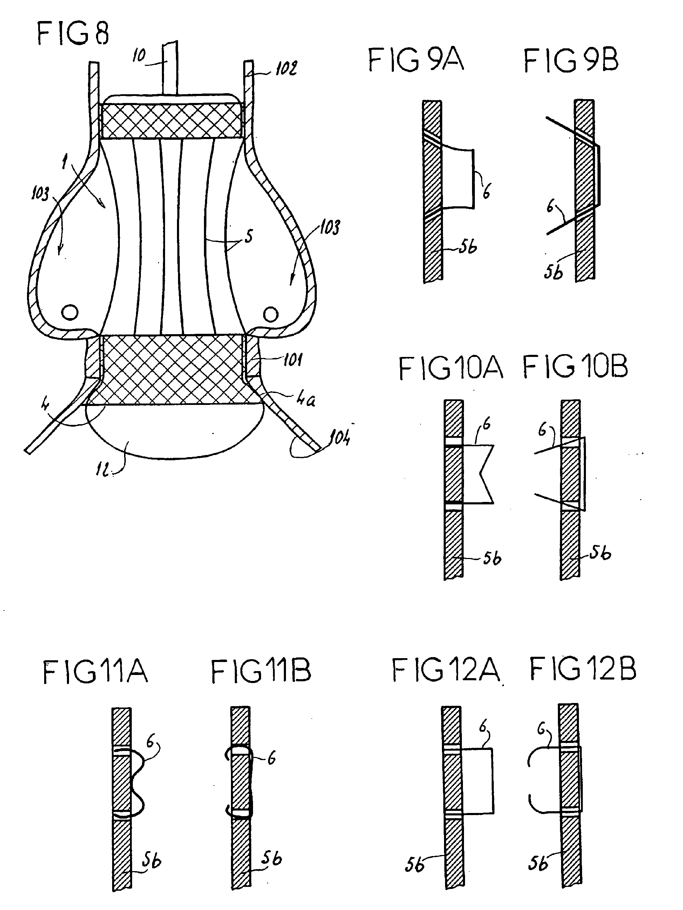

La figure 8 montre un ensemble 1 dans lequel la zone proximale 4a de la portion tubulaire proximale 4 présente, ou adopte par déformation élastique, une forme tronconique adaptée à prendre appui contre la paroi 104 du ventricule. Le ballonnet 12 permet le déploiement de cette zone 4a.

Les figures 9A à 17 montrent différentes variantes possibles dans les formes des palettes 5b et des agrafes 6.

Les figures 9A à 13C montrent des agrafes 6 permettant un ancrage par déformation plastique. Dans ce cas, les trous d'une palette 5b peuvent ne pas être orientés radialement mais en oblique (cf. figure 9A) de manière à réaliser un déploiement oblique des branches d'ancrage de l'agrafe 6 (cf. figure 9B), renforçant la résistance de l'ancrage obtenu ; la branche médiane de l'agrafe 6 peut présenter une forme non rectiligne, soit en chevron (cf. figure 10A) soit ondulée (cf. figure 11 A), pour, lorsque les ballonnets 13, 14 sont appliqués contre elle, orienter les branches d'ancrage selon des directions obliques (cf. figures 10B, 1 1 B) ; les branches latérales peuvent être rectilignes (cf. figures 9A à 10B) ou courbes (cf. figures 1 1 A, 11 B).

Au moins une agrafe 6 peut également être en un matériau à mémoire de forme, notamment l'alliage de nickel et de titane connu sous la dénomination "NITINOL". Les branches d'ancrage de cette agrafe 6 peuvent alors être rectilignes avant ancrage (cf. figure 12A) et peuvent prendre une forme courbe par mémoire de forme (cf. figure 12B).

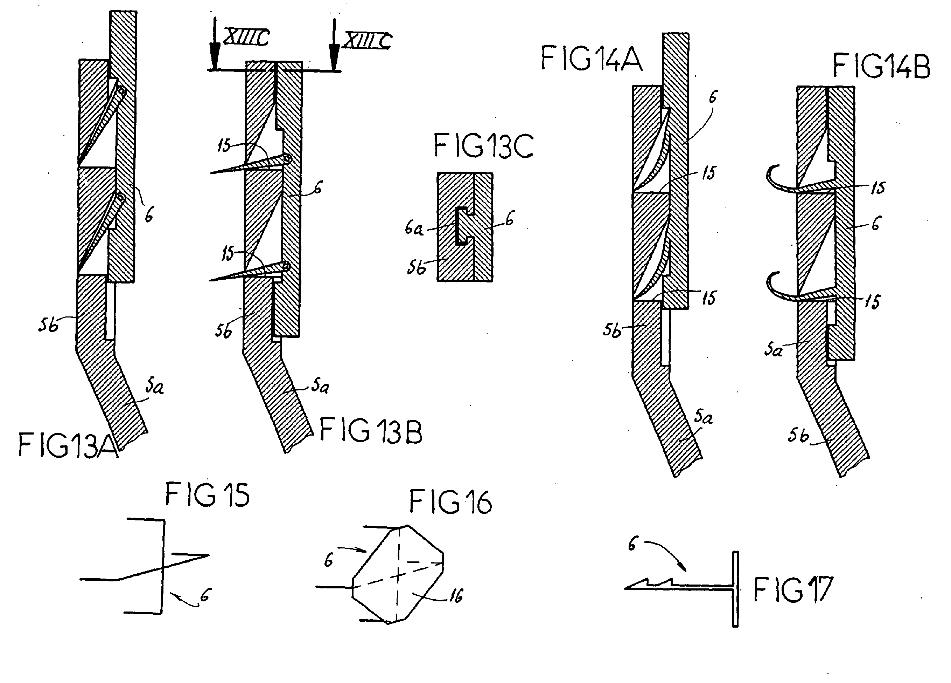

L'agrafe 6 peut également être déplaçable axialement par rapport à la palette 5b, en étant guidée par rapport à celle-ci, pour déployer lesdites branches d'ancrage. Ce guidage peut notamment être réalisé par engagement d'un ou plusieurs patins 6a qu'elle comprend dans une rainure en "T" aménagée dans la palette 5b (cf. figures 13A à 13C) ; ces branches d'ancrage peuvent être pivotantes et être déplacées par prise d'appui contre des parois 15 correspondantes de la palette 5b (cf. figures 13A,13B), ou être déformables (cf. figures 14A, 14B).

Ce déplacement peut notamment être réalisé au moyen d'un ballonnet à plusieurs chambres, gonflées successivement pour pousser axialement les agrafes 6 vers la position de déploiement de leurs branches d'ancrage.

Les figures 15 et 16 montrent que les agrafes 6 peuvent présenter deux branches médianes perpendiculaires et comprendre alors quatre branches d'ancrage favorisant un enfoncement perpendiculaire dans la paroi. Ces branches médianes peuvent comporter une plaquette 16 favorisant la prise d'appui des ballonnets 13, 14 contre elles.

La figure 17 montre que les agrafes 6 peuvent présenter une forme de harpon. Elles peuvent également être en forme de punaises.

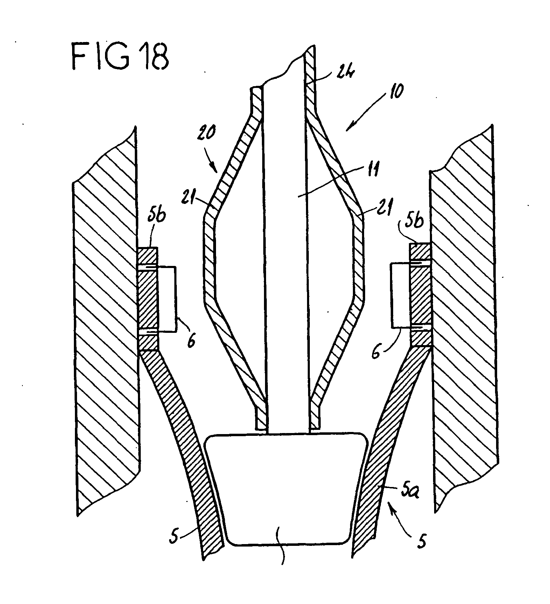

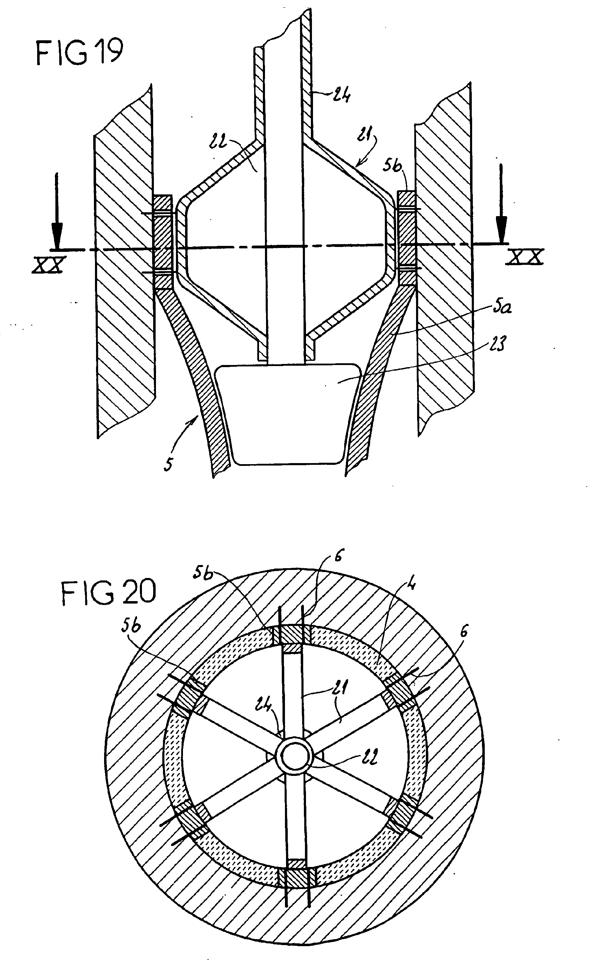

Les figures 18 à 20 montrent que le ballonnet 14 peut être remplacé par des bras radiaux déployables 21. L'instrument 10 présente alors un cathéter 11, un ballonnet 23 de déploiement des éléments 5 et une gaine intérieure 24 reliée par son extrémité proximale au cathéter 11 mais pouvant coulisser par rapport à ce dernier en dehors de cette extrémité. Cette gaine 24 présente des fentes longitudinales qui individualisent les bras 21, et son coulissement provoque le déploiement de ces bras 21 par déformation élastique de cette gaine 24.

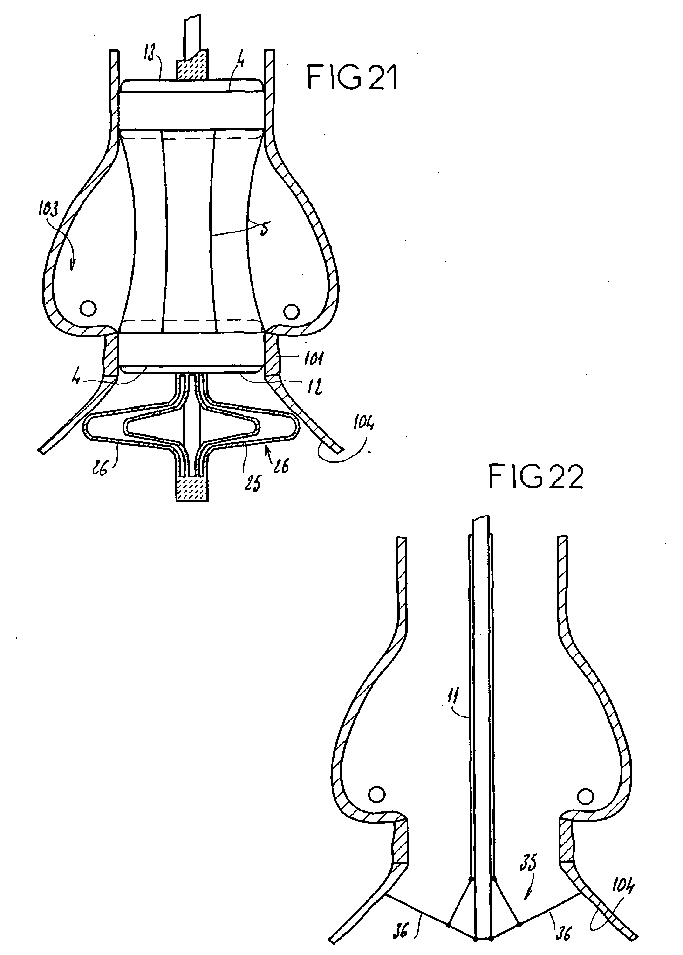

La figure 21 montre que le ballonnet 12 peut être remplacé par une structure 25 à bras radiaux déployables 26 similaires à ceux décrits en référence aux figures 18 à 20.

La figure 22 montre que le ballonnet 12 peut être remplacé par une structure 35 déployable à la manière d'un parapluie, cette structure 35 comprenant une série de "baleines" 36 de prise d'appui contre la paroi 104.

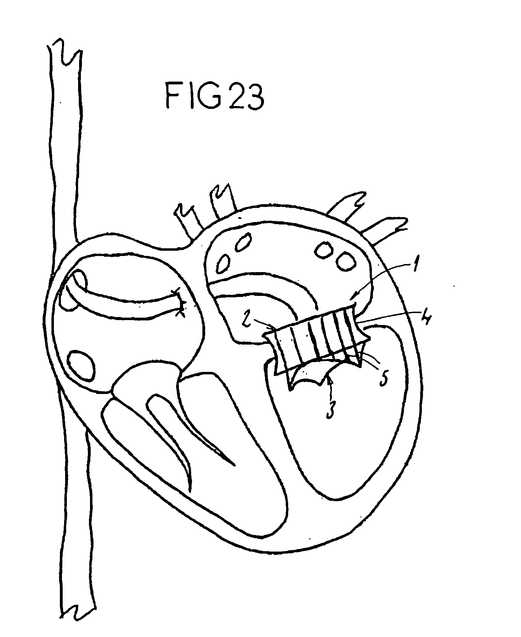

La figure 23 montre un ensemble 1 dont le support 2 comprend une portion tubulaire 4 unique, de laquelle font saillie, sur une extrémité axiale de cette portion tubulaire 4, des éléments allongés d'armature 5. Ces derniers sont rectilignes et de longueur limitée ; ils permettent, de la même manière que précitée, le déploiement de la valve 3.

Ainsi qu'il apparaît de ce qui précède, l'invention fournit un ensemble permettant la mise en place d'une valve prothétique dans un conduit corporel, notamment une valve cardiaque et en particulier une valve aortique, qui présente des avantages déterminants par rapport aux ensembles homologues de la technique antérieure.

Il va de soi que l'invention n'est pas limitée à la forme de réalisation décrite ci-dessus à titre d'exemple mais qu'elle en embrasse au contraire toutes les variantes de réalisation entrant dans le champ de protection défini par les revendications ci-annexées. Ainsi, les éléments 5 peuvent présenter une forme rectiligne au repos et être contraints au niveau de leur partie centrale par un élément bridant leur expansion radialement afin de leur donner une courbure vers l'intérieur du conduit corporel ; le ballonnet 13 peut être remplacé par des bras radiaux déployables ; d'autres moyens de positionnement proximal que le ballonnet 12, les bras 25 ou les baleines 36 peuvent être envisagés ; le cathéter 11 peut comprendre un clapet anti-retour.

## Revendications

1. Ensemble (1) permettant la mise en place d'une valve prothétique (3) dans un conduit corporel, comprenant la valve prothétique (3) à implanter et un support (2) recevant cette valve (3), la valve (3) et le support (2) étant conformés de manière à pouvoir adopter une position de contraction radiale, qui permet leur insertion dans le conduit corporel à équiper à l'aide de moyens (10) d'insertion / déploiement, et une position de déploiement radial, dans laquelle le support (2) est immobilisé par rapport à la paroi du conduit corporel à équiper ; le support (2) comprenant:
- au moins une portion tubulaire (4) en matériau souple faiblement étirable dans le sens circonférentiel, qui présente, dans sa position de déploiement, une dimension dans le sens circonférentiel correspondant sensiblement à la dimension dans le sens circonférentiel de l'emplacement (101, 102) du conduit corporel en regard duquel cette portion tubulaire (4) doit être implantée ;
- des moyens (6) de fixation de cette portion tubulaire (4) à la paroi du conduit corporel, et l'ensemble est caractèrisé en ce que le support comprend:
- une pluralité d'éléments allongés d'armature (5), disposés sur la circonférence de ladite portion tubulaire (4) et reliés à cette portion tubulaire (4) de manière indépendante les uns des autres ; la valve (3) est reliée au moins partiellement à ces éléments allongés d'armature (5), notamment au niveau des commissures (3a) de ses valvules, et ces éléments allongés d'armature (5) forment conjointement, en position déployée, une structure ayant un diamètre prédéterminé qui assure le déploiement adéquat de cette valve (3).

2. Ensemble (1) selon la revendication 1, **caractérisé en ce que** la valve (3) présente un anneau de base permettant sa liaison étanche, notamment par suture, à ladite portion tubulaire (4) ou à l'une desdites portions tubulaires (4).

3. Ensemble (1) selon la revendication 1 ou la revendication 2, pour l'implantation d'une valve aortique (3), **caractérisé en ce que** ladite portion tubulaire (4), ou une paroi périphérique (3b) que comprend la valve (3), présente des ouvertures latérales (18) de manière à permettre un écoulement naturel du sang vers les ostia coronaires (103), sans risque de stagnation.

4. Ensemble (1) selon l'une des revendications 1 à 3, **caractérisé en ce que** la ou lesdites portions tubulaires présentent au moins une chambre périphérique gonflable, pouvant être gonflée de manière à former un joint assurant l'étanchéité entre cette portion tubulaire et la paroi du conduit corporel à équiper.

5. Ensemble (1) selon l'une des revendications 1 à 4, pour l'implantation d'une valve aortique (3), **caractérisé en ce que** la zone proximale de ladite portion tubulaire, ou de la portion tubulaire (4) proximale en cas de pluralité de portions tubulaires, présente une forme tronconique adaptée à prendre appui contre la paroi du ventricule.

6. Ensemble (1) selon l'une des revendications 1 à 5, **caractérisé en ce que** lesdits moyens de fixation sont constitués par des agrafes d'ancrage (6) présentant des branches d'ancrage, ces branches d'ancrage venant s'insérer dans la paroi du conduit corporel à équiper en fin de déploiement de cette ou ces portions tubulaires (4).

7. Ensemble (1) selon la revendication 6, **caractérisé en ce que** les agrafes (6) sont plastiquement déformables et **en ce que** des moyens (5b, 15) sont prévus pour déployer les branches d'ancrage de ces agrafes (6) de manière non radiale, notamment en oblique ou selon une trajectoire courbe.

8. Ensemble (1) selon la revendication 6, **caractérisé en ce qu'**au moins une agrafe (6) est en un matériau à mémoire de forme tel que l'alliage de nickel et de titane connu sous la dénomination "NITINOL".

9. Ensemble (1) selon l'une des revendications 1 à 8, **caractérisé en ce que** lesdits moyens de fixation comprennent une colle biologique.

10. Ensemble (1) selon la revendication 9, **caractérisé en ce que** la colle est contenue dans des vésicules ruptibles placées sur la face radialement externe de la ou des portions tubulaires.

11. Ensemble (1) selon l'une des revendications 1 à 10, **caractérisé en ce que** chaque élément allongé d'armature (5) présente une forme courbe et est disposé avec son côté convexe tourné radialement vers l'intérieur du support (2), de telle sorte que ce support (2) présente, au niveau de sa zone médiane, un diamètre inférieur par rapport à celui qu'il présente au niveau de ses extrémités axiales.

12. Ensemble (1) selon l'une des revendications 1 à 11, **caractérisé en ce que**.le support (2) comprend :
- deux portions tubulaires (4) telles que précitées, et
- l'une des extrémités desdits éléments allongés d'armature (5) est reliée à l'une de ces portions tubulaires (4) tandis que l'autre extrémité de ces éléments allongés d'armature (5) est reliée à l'autre portion tubulaire (4).

13. Ensemble selon l'une des revendications 1 à 11, **caractérisé en ce que** le support comprend une portion tubulaire unique s'étendant sur l'ensemble du support, à la paroi de laquelle sont reliés lesdits éléments allongés d'armature.

14. Ensemble selon l'une des revendications 1 à 11, **caractérisé en ce que** le support (2) comprend une portion tubulaire (4) unique, de laquelle font saillie, sur une extrémité axiale de cette portion tubulaire (4), lesdits éléments allongés d'armature (5).

15. Ensemble selon l'une des revendications 1 à 14, **caractérisé en ce que** les éléments allongés d'armature présentent une forme rectiligne au repos et sont contraints au niveau de leur partie centrale par un élément bridant leur expansion radialement afin de leur donner une courbure vers l'intérieur du conduit corporel.

16. Combinaison de l'ensemble (1) selon l'une des revendications 12 à 15 et de moyens (10) d'insertion/déploiement pour la mise en place de cet ensemble (1), **caractérisée en ce que** lesdits moyens d'insertion/déploiement comprennent un cathéter (11) d'écoulement du sang le temps de l'implantation de l'ensemble (1), au moins un ballonnet (13, 14) de déploiement d'au moins une portion tubulaire (4) et une gaine (9) de maintien de cette portion tubulaire (4) et des éléments allongés d'armature (5) dans une position de contraction.

17. Combinaison selon la revendication 16, **caractérisée en ce que** lesdits moyens (10) d'insertion/déploiement comprennent un moyen proximal (12, 25, 36) de positionnement, dimensionné pour prendre appui contre la paroi (104) du ventricule.

18. Combinaison selon la revendication 16 ou la revendication 17, **caractérisée en ce que** lesdits moyens (10) d'insertion/déploiement comprennent une pompe facilitant l'écoulement du sang au-delà de l'ensemble (1) le temps de l'implantation.

## Claims

1. An assembly (1) allowing the setting of a valve prosthesis (3) in a corporeal duct, comprising the valve prosthesis (3) to implant and a support (2) receiving this valve (3), the valve (3) and the support (2) being shaped in such a way as to be able to adopt a radial contraction position, which allows their insertion in the corporeal duct to be equipped with the help of insertion/extension means (10), and a radial extension position, in which the support (2) is immobilized with respect to the wall of the corporeal duct to be equipped; the support (2) comprising:
- at least one tubular portion (4) in a pliable material which is slightly stretchable in the circumferential direction, which presents, in its extension position, a dimension in the circumferential direction roughly corresponding to the dimension in the circumferential direction of the location (101, 102) of the corporeal duct in regards to which this tubular portion (4) must be implanted;
- means (6) for fixing this tubular portion (4) to the wall of the corporeal duct, and the assembly is **characterized in that** the support comprises:
- a plurality of elongated reinforcing elements (5), placed on the circumference of said tubular portion (4) and linked to this tubular portion (4) so that they are independent one from the others; the valve (3) is linked at least partially to these elongated reinforcing elements (5), particularly at the commissures (3a) of these valvelets, and these elongated reinforcing elements (5) jointly form, in extended position, a structure having a predetermined diameter which ensures sufficient extension of this valve (3).

2. The assembly (1) according to claim 1, **characterized in that** the valve (3) presents a base ring allowing the tight connection, particularly by suturing, to said tubular portion (4) or to one of said tubular portions (4).

3. The assembly (1) according to claim 1 or claim 2, for implanting an aortic valve (3), **characterized in that** said tubular portion (4), or a peripheral wall (3b) which comprises the valve (3), presents side openings (18) so that a natural flow of blood to the coronary ostia (103) is allowed, without risk of stagnation.

4. The assembly (1) according to any of claims 1 to 3, **characterized in that** said tubular portion or portions present(s) at least one inflatable peripheral chamber, which may be inflated so to form a joint ensuring the sealing between this tubular portion and the wall of the corporeal duct to be equipped.

5. The assembly (1) according to any of claims 1 to 4, for implanting an aortic valve (3), **characterized in that** the proximal zone of said tubular portion, or of the proximal tubular portion (4) in case of a plurality of tubular portions, presents a truncated cone shape suitable for bearing against the wall of the ventricle.

6. The assembly (1) according to any of claims 1 to 5, **characterized in that** said fixation means are constituted of anchoring fasteners (6) presenting anchoring prongs, these anchoring prongs are inserted into the wall of the corporeal duct to be equipped at the end of the extension of this or these tubular portions (4).

7. The assembly (1) according to claim 6, **characterized in that** the fasteners (6) are plastically deformable and **in that** the means (5b, 15) are provided for extending the anchoring prongs of these fasteners (6) in a non-radial way, particularly in an oblique orientation or according to a trajectory curve.

8. The assembly (1) according to claim 6, **characterized in that** at least one fastener (6) is in a shape-retaining material such as the nickel and titanium alloy known under the name "NITINOL."

9. The assembly (1) according to any of claims 1 to 8, **characterized in that** said fixation means comprise a biological glue.

10. The assembly (1) according to claim 9, **characterized in that** the glue is contained in breakable blisters placed on the radially external surface of the tubular portion or portions.

11. The assembly (1) according to any of claims 1 to 10, **characterized in that** each elongated reinforcing element (5) presents a curved form and is placed with its convex side radially turned toward the inside of the support (2), so that the support (2) presents, at its median zone, a smaller diameter compared to that present at its axial extremities.

12. The assembly (1) according to any of claims 1 to 11, **characterized in that** the support (2) comprises:
- two tubular portions (4) such as the aforementioned, and
- one of the extremities of said elongated reinforcing elements (5) is linked to one of these tubular portions (4) while the other extremity of these elongated reinforcing elements (5) is linked to the other tubular portion (4).

13. The assembly according to any of claims 1 to 11, **characterized in that** the support comprises a single tubular portion stretching on the support assembly, to the wall of which are linked said elongated reinforcing elements.

14. The assembly according to any of claims 1 to 11, **characterized in that** the support (2) comprises a single tubular portion (4), from which said elongated reinforcing elements (5) protrude on an axial extremity of this tubular portion (4).

15. The assembly according to any of claims 1 to 14, **characterized in that** the elongated reinforcing elements present a rectilinear form at rest and are limited to their central part by an element clamping their radial expansion in order to give them a curve toward the inside of the corporeal duct.

16. Combination of the assembly (1) according to any of claims 12 to 15 and of insertion/extension means (10) for setting the assembly (1), **characterized in that** said insertion/extension means comprise a blood flow catheter (11) for the time of implanting the assembly (1), at least one balloon (13, 14) for extending at least one tubular portion (4) and a sheath (9) for maintaining this tubular portion (4) and the elongated reinforcing elements (5) in a contraction position.

17. Combination according to claim 16, **characterized in that** said insertion/extension means (10) comprise a proximal positioning means (12, 25, 36), dimensioned for bearing against the wall (104) of the ventricle.

18. Combination according to claim 16 or claim 17, **characterized in that** said insertion/extension means (10) comprise a pump facilitating the flow of blood beyond the assembly (1) at the time of implantation.

## Patentansprüche

1. Gruppe (1) zum Einsetzen eines prothetischen Ventils (3) in eine Körperleitung, die das zu implantierende prothetische Ventil (3) und eine Basis (2) umfasst, die dieses Ventil (3) aufnimmt, wobei das Ventil (3) und die Basis (2) derart ausgebildet sind, dass sie eine radiale Kontraktionsstellung einnehmen können, die ihr Einsetzen in die auszustattende Körperleitung mit Hilfe von Einfüge-/Entfaltungsmitteln (10) erlaubt, und eine radiale Entfaltungsstellung, in welcher die Basis (2) in Bezug auf die Wand der auszustattenden Körperleitung immobilisiert ist,
wobei die Basis (2) umfasst
- mindestens einen röhrenförmigen Abschnitt (4) aus einem elastischen, schwach in Umfangsrichtung auseinanderziehbaren Material, der in seiner entfalteten Stellung in Umfangsrichtung eine Größe aufweist, die in Umfangsrichtung etwa der Größe der Stelle (101, 102) der Körperleitung entspricht, der gegenüber dieser röhrenförmigen Abschnitt (4) zu implantieren ist,
- Mittel (6) zur Befestigung dieses röhrenförmigen Abschnitts (4) an der Wand der Körperleitung, und die Gruppe ist **dadurch gekennzeichnet, dass** die Basis umfasst:
- eine Vielzahl verlängerter Gestellelemente (5), die auf dem Umfang des röhrenförmigen Abschnitts (4) angeordnet und mit diesem röhrenförmigen Abschnitt (4) unabhängig voneinander verbunden sind, das Ventil (3) ist mindestens teilweise mit diesen verlängerten Gestellelementen (5) verbunden, vor allem auf Ebene der Winkel (3a) seiner Klappen, und diese verlängerten Gestellelemente (5) bilden zusammen in entfalteter Stellung eine Struktur mit einem vorbestimmten Durchmesser, der die adäquate Entfaltung dieses Ventils (3) sicherstellt.

2. Gruppe (1) nach Anspruch 1, **dadurch gekennzeichnet, dass** das Ventil (3) einen Basisring aufweist, der seine dichte Verbindung, vor allem durch Nähen, mit dem röhrenförmigen Abschnitt (4) oder einem der röhrenförmigen Abschnitte (4) erlaubt.

3. Gruppe (1) nach Anspruch 1 oder Anspruch 2 zur Implantation eines Aortaventils (3), **dadurch gekennzeichnet, dass** der röhrenförmige Abschnitt (4) oder eine periphere Wand (3b), die das Ventil (3) umfasst, seitliche Öffnungen (18) aufweist, so dass ein natürlicher Blutfluss in Richtung der Koronarostien (103) ohne Stillstandsgefahr erlaubt wird.

4. Gruppe (1) nach einem der Ansprüche 1 bis 3, **dadurch gekennzeichnet, dass** der oder die röhrenförmigen Abschnitte mindestens eine aufblasbare periphere Kammer aufweisen, die derart aufblasbar ist, dass sie eine Dichtung bildet, die die Dichtigkeit zwischen diesem röhrenförmigen Abschnitt und der Wand der auszustattenden Körperleitung bildet.

5. Gruppe (1) nach einem der Ansprüche 1 bis 4 zur Implantation eines Aortaventils (3), **dadurch gekennzeichnet, dass** der proximale Bereich des röhrenförmigen Abschnitts oder des proximalen röhrenförmigen Abschnitts (4) bei einer Vielzahl röhrenförmiger Abschnitte eine Kegelstumpfform aufweist, die geeignet ist, um sich auf der Wand der Herzkammer abzustützen.

6. Gruppe (1) nach einem der Ansprüche 1 bis 5, **dadurch gekennzeichnet, dass** die Befestigungsmittel von Verankerungsklammern (6) gebildet werden, die Verankerungsschenkel aufweisen, wobei diese Verankerungsschenkel am Ende der Entfaltung dieses oder dieser röhrenförmigen Abschnitte (4) in die Wand der auszustattenden Körperleitung eingreifen.

7. Gruppe (1) nach Anspruch 6, **dadurch gekennzeichnet, dass** die Klammern (6) plastisch deformierbar sind und **dadurch**, dass Mittel (5b, 15) vorgesehen sind, um die Verankerungsschenkel dieser Klammern (6) nicht radial, vor allem schräg oder gemäß einer gekrümmten Bahn, zu entfalten.

8. Gruppe (1) nach Anspruch 6, **dadurch gekennzeichnet, dass** mindestens eine Klammer (6) aus einem Material mit Formengedächtnis ist, wie zum Beispiel die unter der Bezeichnung NITINOL bekannte Nickel- und Titanlegierung.

9. Gruppe (1) nach einem der Ansprüche 1 bis 8, **dadurch gekennzeichnet, dass** die Befestigungsmittel einen biologischen Klebstoff umfassen.

10. Gruppe (1) nach Anspruch 9, **dadurch gekennzeichnet, dass** sich der Klebstoff in aufbrechbaren Blasen befindet, die auf der radial externen Seite des oder der röhrenförmigen Abschnitte platziert sind.

11. Gruppe (1) nach einem der Ansprüche 1 bis 10, **dadurch gekennzeichnet, dass** jedes verlängerte Gestellelement (5) eine gekrümmte Form aufweist und mit seiner konvexen Seite radial in Richtung Innenraum der Basis (2) gedreht angeordnet ist, so dass diese Basis (2) auf Ebene ihres medianen Bereichs einen Durchmesser aufweist, der in Bezug auf den Durchmesser, den sie auf Ebene ihrer axialen Enden aufweist, kleiner ist.

12. Gruppe (1) nach einem der Ansprüche 1 bis 11, **dadurch gekennzeichnet, dass** die Basis (2) umfasst:
- zwei vorgenannte röhrenförmige Abschnitte (4) und
- eines der Enden der verlängerten Gestellelemente (5) ist mit einem dieser röhrenförmigen Abschnitte (4) verbunden, wogegen das andere Ende dieser verlängerten Gestellelemente (5) mit dem anderen röhrenförmigen Abschnitt (4) verbunden ist.

13. Gruppe nach einem der Ansprüche 1 bis 11, **dadurch gekennzeichnet, dass** die Basis einen einzigen röhrenförmigen Abschnitt umfasst, der sich über die gesamte Basis erstreckt und mit dessen Wand die verlängerten Gestellelemente verbunden sind.

14. Gruppe nach einem der Ansprüche 1 bis 11, **dadurch gekennzeichnet, dass** die Basis (2) einen einzigen röhrenförmigen Abschnitt (4) umfasst, über den, über ein axiales Ende dieses röhrenförmigen Abschnitts (4), die verlängerten Gestellelemente (5) hinausragen.

15. Gruppe nach einem der Ansprüche 1 bis 14, **dadurch gekennzeichnet, dass** die verlängerten Gestellelemente in Ruhe eine gerade Form aufweisen und auf Ebene ihres mittigen Teils durch ein Element gespannt sind, das ihre Expansion radial flanscht, um ihnen eine Krümmung in Richtung Innenraum der Körperleitung zu verleihen.

16. Kombination der Gruppe (1) nach einem der Ansprüche 12 bis 15 und der Einfüge-/Entfaltungsmittel (10) zur Platzierung dieser Gruppe (1), **dadurch gekennzeichnet, dass** die Einfüge-/Entfaltungsmittel einen Blutflusskatheter (11) für die Zeit der Implantation der Gruppe (1) umfassen, mindestens einen Entfaltungsballon (13, 14) mindestens eines röhrenförmigen Abschnitts (4) und eine Hülle (9) zum Halten dieses röhrenförmigen Abschnitts (4) und der verlängerten Gestellelemente (5) in einer Kontraktionsstellung.

17. Kombination nach Anspruch 16, **dadurch gekennzeichnet, dass** die Einfüge-/Entfaltungsmittel (10) ein proximales Positionierungsmittel (12, 25, 36) umfassen mit einer Größe, um sich auf der Wand (104) der Herzkammer abzustützen.

18. Kombination nach Anspruch 16 oder Anspruch 17, **dadurch gekennzeichnet, dass** die Einfüge-/Entfaltungsmittel (10) eine Pumpe umfassen, die das Fließen des Bluts jenseits der Gruppe (1) während der Implantationszeit erleichtert.
